(19) Europäisches Patentamt
European Patent Office
Office européen des brevets

(11) Publication number : 0 428 107 B1

(12) # EUROPEAN PATENT SPECIFICATION

(45) Date of publication of patent specification : **19.01.94 Bulletin 94/03**

(21) Application number : **90121634.1**

(22) Date of filing : **12.11.90**

(51) Int. Cl.⁵ : **A61K 31/47,** A61K 31/71, A61K 31/475, A61K 37/02, // (A61K31/71, 31:47), (A61K31/475, 31:47), (A61K37/02, 31:47)

(54) **Use of quinolyl-and isoquinolyloxanole-2-ones for the production of a medicament for the treatment of multi-drug resistant tumors.**

(30) Priority : **13.11.89 US 436262**

(43) Date of publication of application : **22.05.91 Bulletin 91/21**

(45) Publication of the grant of the patent : **19.01.94 Bulletin 94/03**

(84) Designated Contracting States : **AT BE CH DE DK ES FR GB GR IT LI LU NL SE**

(56) References cited :
**EP-A- 0 359 981**
**EP-A- 0 365 863**
**H.M. PINEDO et al.: "Cancer Chemotherapy and Biological Response Modifiers", vol. 10, 1988, pages 73-84; R.L. FINE: "Multidrug resistance"**
**Fed. Am. Soc. Exp. Biol. 73rd Ann. Meeting, 19-23, March 1989, vol. 3, pag. 1, Abstr. no. 1230, H.C. Cheng et al.**

(73) Proprietor : **MERRELL DOW PHARMACEUTICALS INC.**
**P.O. Box 156300 2110 East Galbraith Road**
**Cincinnati Ohio 45215-6300 (US)**

(72) Inventor : **Sunkara, Sai P.**
**9361 Bluewing Terrace**
**Cincinnati, Ohio 45236 (US)**
Inventor : **Jones, Winton D.**
**1464 Longacre Drive**
**Cincinnati, Ohio 45240 (US)**

(74) Representative : **VOSSIUS & PARTNER**
**Postfach 86 07 67**
**D-81634 München (DE)**

EP 0 428 107 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of certain quinolyl- and isoquinolyloxazole-2-ones in the treatment of multi-drug resistant tumors.

Effective tumor treatment is frequently thwarted by the lack of sensitivity of certain tumors to standard chemotherapeutic agents (intrinsic resistance) or by the ability of certain tumors to develop a lack of chemotherapeutic sensitivity during the course of treatment (acquired resistance). The cause of this phenomenon has, at least in part, been demonstrated to result from the existance of an energy-dependent efflux pump which acts to remove the chemotherapeutic agent from the target cell.

The pump consists of P-glycoprotein found as a constituent of cell membrane, and it has been suggested that the normal function of P-glycoprotein is to remove toxins from within the cell. This theory is supported by the observation that P-glycoprotein is found as a cell membrane constitutent in cells such as liver, kidney, colon, and jejunum. It has been suggested that P-glycoprotein in the cell membrane of such normal tissues could act to remove toxins or to assist in the transport of nutrients and solutes and in secreting a variety of protein and steroid substances. Natural presence of P-glycoprotein in tumor cells derived from these tissues as well as its presence in tumor cells derived from other tissue types could explain, at least in part, resistance of various tumors to therapy with standard chemotherapeutic agents.

The use of therapeutic agents which inactivate the P-glycoprotein pump would be invaluable in the treatment of multidrug-resistant tumors. Quinidine and reserpine as well as the calcium channel blockers verapamil and diltiazem have been reported to reverse drug resistance in multidrug-resistant tumors. Such agents could function by, for example, interfering with transcription of the P-glycoprotein gene, blocking the drug binding site on the P-glycoprotein or by decoupling the energy dependent driving mechanism of the efflux pump.

Applicants have determined that certain quinolyl- and isoquinolyloxazole-2-ones having PKC inhibiting activity are useful in the treatment of multi-drug resistant tumors. The quinolyl- and isoquinolyloxazolone-2-ones of this invention act to reverse drug resistance and therby allow standard chemotherapeutic agents to exhibit normal toxicity on tumors.

The present invention is directed to the use of certain quinolyl- and isoquinolyloxazole-2-ones of the formula

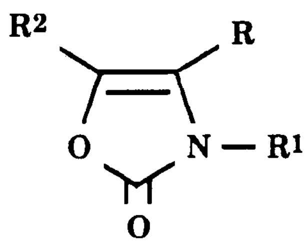

(I)

wherein

R and $R^1$ are each independently selected from hydrogen, $C_1$-$C_6$ alkyl, and phenyl or $C_1$-$C_3$ alkylphenyl wherein the phenyl ring is optionally substituted with one, two or three of the substituents selected from fluorine, chlorine, bromine, $C_1$-$C_4$ alkyl, and $C_1$-$C_4$ alkoxy; and

$R^2$ is a 2-, 3-, or 4-quinolyl group or a 1-, 3-, or 4-isoquinolyl group wherein the quinolyl or isoquinolyl group is optionally substituted with one, two or three of the substituents selected from fluorine, chlorine, bromine, $C_1$-$C_4$ alkyl, $C_1$-$C_4$ alkoxy, nitro, trifluoromethyl, or phenyl wherein the phenyl is optionally substituted by fluorine, chlorine, bromine, $C_1$-$C_4$ alkyl, or $C_1$-$C_4$ alkoxy; or $R^2$ is a 5-, 6-, 7-, or 8-quinolyl or isoquinolyl group;

and the pharmaceutically-acceptable salts thereof in the treatment of multidrug-resistant tumors.

This invention concerns the use of the compounds of Formula I as agents effective in the treatment of multi-drug resistant tumors. Specifically the compounds of formula I, when administered together with standard chemotherapeutic agents, can be used in the treatment of tumors which are intrinsically or extrinsically drug resistant.

As used herein, the terms "$C_1$-$C_3$ alkyl", "$C_1$-$C_4$ alkyl" and "$C_1$-$C_6$ alkyl" mean straight or branched chain alkyl groups having from one to three, from one to four, or from one to six carbon atoms respectively, and include such groups as methyl, ethyl, n-propyl, isopropyl, n-butyl, isobutyl, sec-butyl and tert-butyl, as well as vinyl, allyl, propynyl, butenyl, butadienyl and isopropenyl. The term "$C_1$-$C_4$ alkoxy" means alkoxy groups having from one to four carbon atoms, and includes such groups as methoxy, ethoxy, n-propoxy, isopropoxy, n-butoxy, isobutoxy, sec-butoxy and tert-butoxy. When R or $R^1$ is "optionally substituted phenyl or $C_1$-$C_3$ alkylphenyl", the one, two or three substituent(s) can be located at any available position on the phenyl ring. When $R^2$ is 2-, 3-, or 4-quinolyl or 1-, 3-, or 4-isoquinolyl the optional substituent(s) can be located at any available position(s) on the quinolyl or isoquinolyl ring.

The expression "a pharmaceutically acceptable acid addition salt" is intended to apply to any non-toxic organic or inorganic acid addition salt of the base compounds. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono, di, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. These salts and the base compounds can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are crystalline materials which are soluble in water and various hydrophilic organic solvents and which in comparison to their free base forms, demonstrate higher melting points and an increased solubility.

Illustrative examples of the compounds used according to this invention include compounds of Formula I wherein the R groups are designated as follows:

| R | $R^1$ | $R^2$ |
|---|---|---|
| hydrogen | hydrogen | 2-, 3-, or 4-quinolyl |
| ethyl | hydrogen | 2-, 3-, or 4-quinolyl |
| propyl | hydrogen | 5-, 6-, 7-, or 8-quinolyl |
| methyl | benzyl | 2-, 3-, or 4-quinolyl |
| phenethyl | hydrogen | 2-, 3-, or 4-quinolyl |
| propyl | hydrogen | 2-, 3-, or 4-(6,7-dimethyl)-quinolyl |
| propyl | hydrogen | 2-, 3-, or 4-(6-phenyl)-quinolyl |
| 4-methoxyphenethyl | hydrogen | 2, 3-, or 4-quinolyl |
| benzyl | benzyl | 2-, 3-, or 4-(7-ethoxy)-quinolyl |

| R | $R^1$ | $R^2$ |
|---|---|---|
| benzyl | benzyl | 2-, 3-, or 4-(7-phenyl)-quinolyl |
| butyl | hydrogen | 2-, 3-, or 4-quinolyl |
| 3,5-dichloro)phenylpropyl | methyl | 5-, 6-, 7-, or 8-quinolyl |
| propyl | methyl | 2-, 3-, or 4-quinolyl |
| 3,5-dimethoxybenzyl | ethyl | 5-, 6-, 7-, or 8 quinolyl |
| methyl | propyl | 2-, 3-, or 4-(5-ethoxy-7-methyl)-quinolyl |
| methyl | propyl | 2-, 3-, or 4-(5-phenyl)-quinolyl |
| butyl | butyl | 5-, 6-, 7-, or 8-quinolyl |
| hydrogen | phenethyl | 2-, 3-, or 4-(6-trifluoromethyl)-quinolyl |
| hydrogen | phenethyl | 2-, 3-, or 4-(6-phenyl)-quinolyl |
| methyl | 4-methoxy-phenethyl | 2-, 3-, or 4-quinolyl |

Equivalently substituted isoquinolyl derivatives are also intended. As is true for most classes of therapeutically effective compounds, certain subclasses and certain species which are especially effective are preferred over others. In this instance, those compounds of Formula I wherein $R^2$ is optionally substituted 2-, 3-, or 4-quinolyl are preferred. Also preferred are compounds wherein R is $C_1$-$C_6$ alkyl, as well as compounds wherein $R^1$ is hydrogen. Most preferred are the compounds wherein $R^2$ is an unsubstituted 2-, 3-, or 4-quinolyl group, R is ethyl or propyl and $R^1$ is hydrogen.

The 2-, 3-, or 4-quinolyloxazole-2-ones of this invention can readily be prepared by the reaction depicted in Reaction Scheme 1.

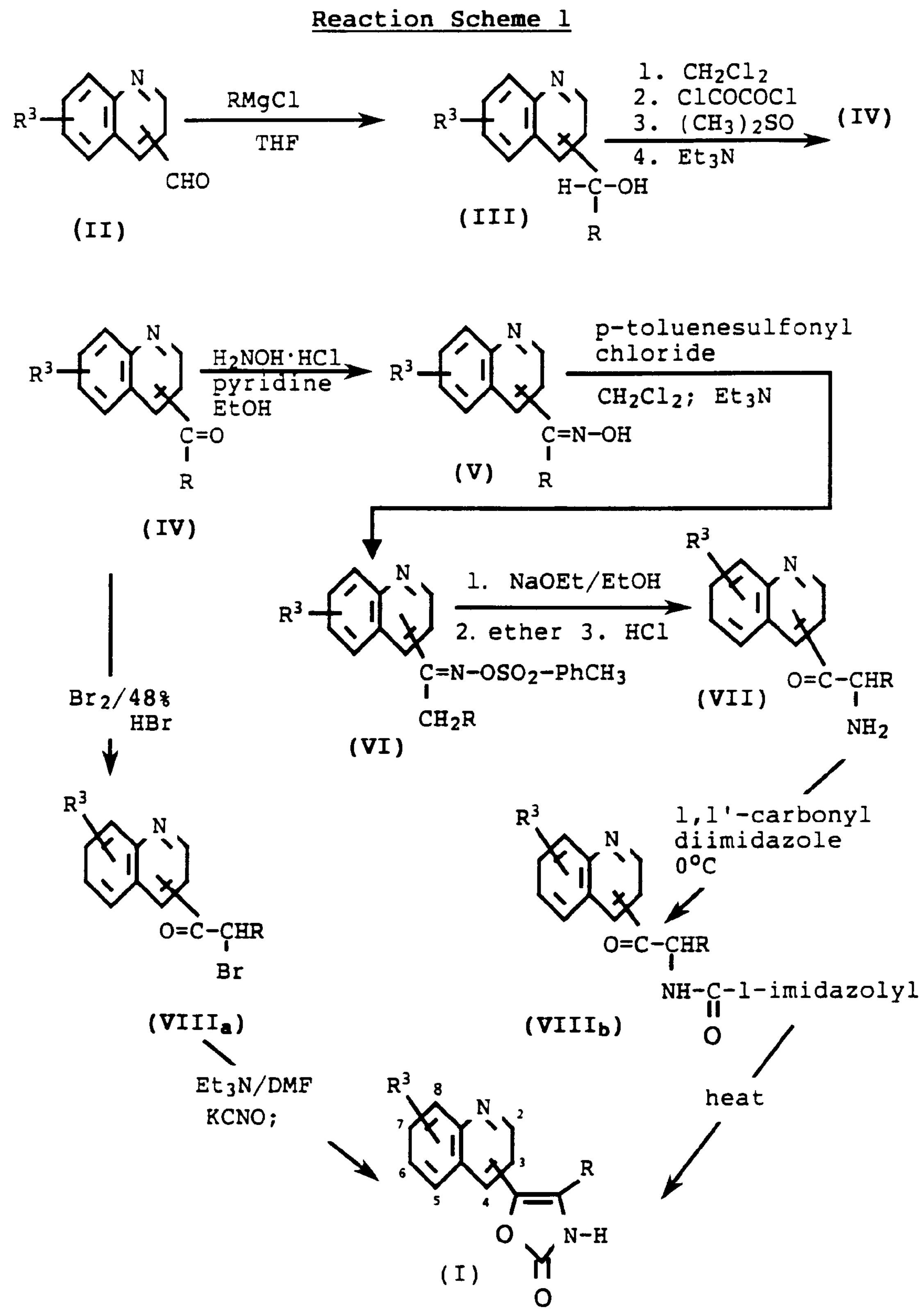


wherein R is as in Formula I, $R^3$ is the optional $R^2$ group substituent(s) of Formula I, and other symbols are as defined hereinafter.

In essence, Reaction Scheme 1 illustrates that the 2-, 3-, or 4-quinolyloxazole-2-ones of Formula I can be prepared by reacting the appropriate and readily available 2-, 3-, or 4-quinoline carboxaldehyde (II) in tetrahydrofuran (THF) with alkylmagnesium chloride or with optionally substituted phenylalkyl-magnesium chloride [RMgCl] to produce 2-, 3-, or 4-quinoline alkanol (III), which is in turn oxidized with oxalyl chloride (ClCO-COCl), methyl sulfoxide [$(CH_3)_2SO$] and triethylamine ($Et_3N$) in dichloromethane ($CH_2Cl_2$) to produce quinolyl-alkanone (IV). The alkanone (IV) can alternately be brominated to compound ($VIII_a$) and further treated with triethylamine in dimethylformamide (DMF) in the presence of potassium cyanate (KCNO) to form the compounds of Formula I according to procedures well known in the art and illustrated in the examples herein; or compound IV can be converted to oxime (V) by refluxing with hydroxylamine hydrochloride ($H_2NOH{\cdot}HCl$) and pyridine in ethanol (EtOH). Compound (V) is then reacted with p-toluenesulfonyl chloride and triethylamine in dichloromethane to produce compound (VI). The amine (VII) is then produced by reacting compound (VI) with sodium ethoxide in ethanol (NaOEt/EtOH), followed by ether and aqueous hydrochloric acid (HCl) extraction. The amine (VII) is further reacted with 1,1'-carbonyldiimidazole at 0°C to form compound ($VIII_b$), which is then heated to 170°C to yield the appropriate 2-, 3-, or 4-quinolyloxazole-2-ones of Formula I. The unsubstituted 5-, 6-, 7- or 8-quinolyloxazole-2-ones of this invention can readily be prepared by the reaction depicted in Reaction Scheme 2.wherein R is as in Formula I, $R^3$ is the optional $R^2$ group substituent of Formula I, and other symbols are as defined for Reaction Scheme 1.

The unsubstituted 5-, 6-, 7- or 8-quinolyloxazole-2-ones of this invention can readily be prepared by the reaction depicted in Reaction Scheme 2.

## Reaction Scheme 2

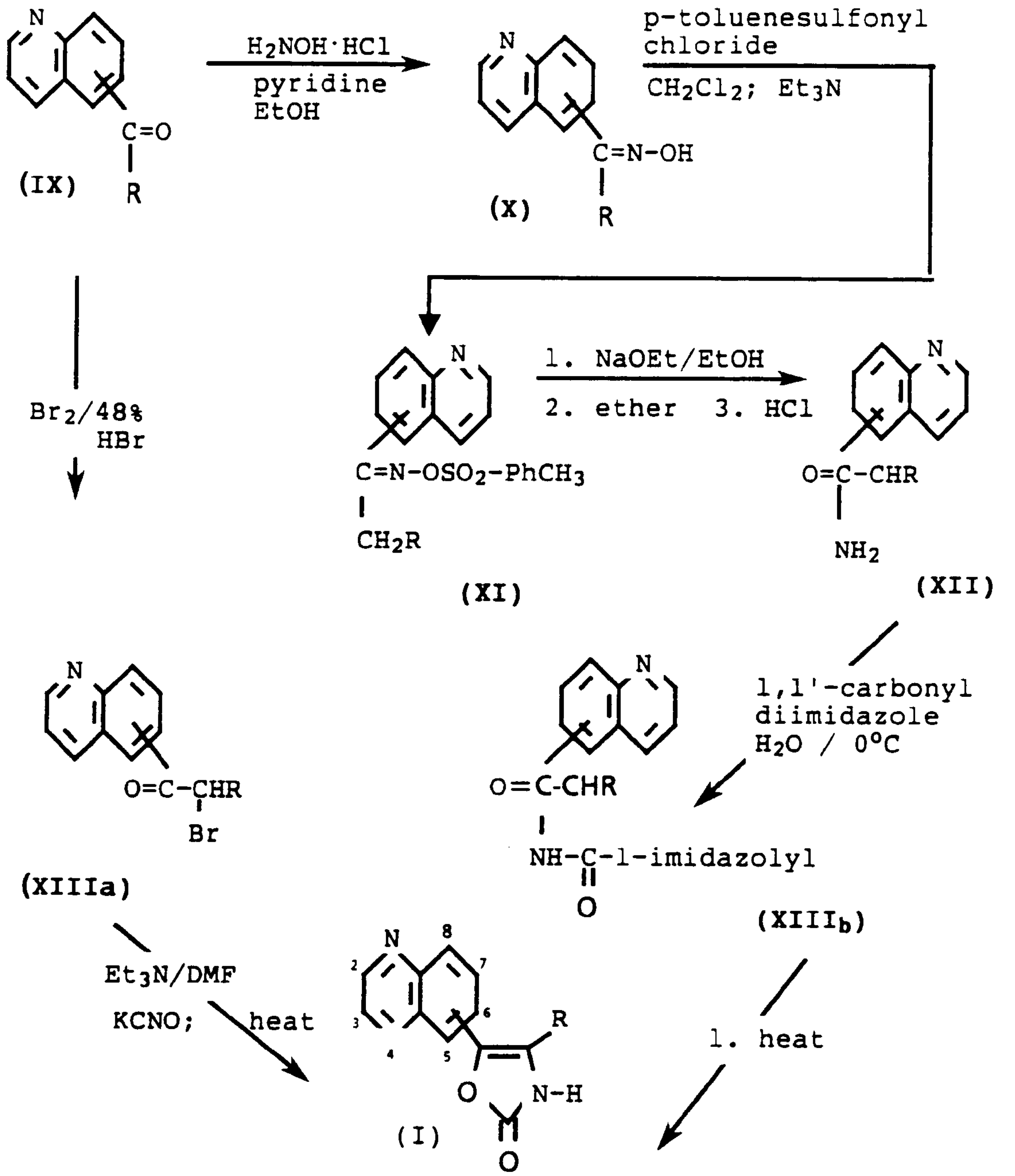


wherein R is as in Formula I, $R^3$ is the optional $R^2$ group substituent of Formula I, and other symbols are as defined for Reaction Scheme 1.

In essence, Reaction Scheme 2 illustrates that the 5-, 6-, 7-, or 8-quinolyloxazole-2-ones of Formula I can be prepared in essentially the same manner as described for Reaction Scheme 1. The alkanone starting material (IX) is prepared by metalating 5-, 6-, 7- or 8-bromoquinoline according to a procedure by H. Gilman and T. Suddy set forth in J. Org. Chem. 23, 1584-9 (1958), and then reacting it with N-alkoxy-N-alkylamine. The 5-, 6-, 7-, or 8-bromoquinoline compounds are prepared by following procedures set forth in "The Chemistry of Heterocyclic Compounds" by Gurnos Jones, as found in Quinolines, Part 1, vol. 32, p. 100-117 and 247-258, ed. A. Weissberger and E.C. Taylor, John Wiley and Sons, London, 1977. These procedures can also be utilized for preparing 2-,3-, or 4-bromoquinolines and their corresponding 2-, 3-, or 4-quinolinyl alkanones such as those of formula (IV) in Reaction Scheme 1.

Alternatively, the formula (IV) and formula (IX) compounds of Reaction Schemes 1 or 2 can also be prepared by reacting the appropriate bromoquinoline with butyl lithium in an appropriate solvent such as THF or

diethyl ether at -70°C to 0°C, preferably at -50°C, and then reacting the lithiated compound with

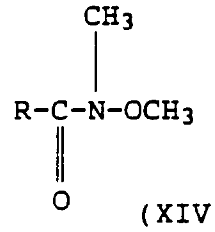

(XIV)

wherein R is as described in Formula I. This reaction is further specifically exemplified in Example 8. Compound (XIV) can be prepared by a procedure set forth in Tetrahedron Letters, 22, 3815 (1981).

The compounds wherein $R^1$ is $C_1$-$C_6$ alkyl or optionally substituted $C_1$-$C_3$ alkylphenyl are produced by subsequent reaction of the compound of Formula I of either Reaction Scheme 1 or Reaction Scheme 2 with sodium hydride and the appropriate alkyl iodide or phenylalkyl iodide in tetrahydrofuran according to procedures well known in the art.

Likewise, one of ordinary skill will readily be able to modify these procedures to obtain isoquinoline derivatives of formula 1.

The ability of the oxazolone derivatives used according to this invention to reverse drug resistance in multidrug resistant tumors can be demonstrated by the ability of test compounds to reduce a cell growth in vinblastine (VBL) resistant tumor cell line.

$CHO^R$ cells were plated at a density of $1X10^5$/35mm dish and were allowed to grow overnight at 37°C in a $CO_2$ incubator. The medium was replaced with medium containing the compounds and vinblastine (0.2 μg/ml). The cells were allowed to grow for further 72 hr and the cell number was determined by Coulter counter after trypsiunization. VLB alone at 0.2 μg/ml did not have any effect on cell growth. The results of such a study employing 4-propyl-5-(4-quinolinyl)-2(3H)-oxazolone are tabulated in Table 1.

TABLE 1

REVERSAL OF MULTIDRUG RESISTANCE (MDR) IN $CHO^R$ CELLS BY 4-PROPYL-5-(4-QUINOLINYL)-2(3H)-OXAZOLONE

| Concentration (μg/ml) | % Inhibition of Cell Growth | |
|---|---|---|
| | Compound Only | Compound + VLB |
| 10 | 14 | 93 |
| 5 | 0 | 93 |
| 1 | 0 | 43 |
| 0.1 | 0 | 4 |

The term "patient" used herein is taken to mean mammals such as primates, including humans, sheep, horses, cattle, pigs, dogs, cats, rats and mice.

The amount of the oxazolone derivative of formula 1 to be administered can vary widely according to the particular dosage unit employed, the period of treatment, the age and sex of the patient treated, the nature and extent of the drug resistance in the tumor to be treated, and the particular oxazolone derivative selected. The oxazolone derivative is used in conjunction with other chemotherapeutic agents known to be useful in the treatment of tumors. The amount of a oxazolone derivative of formula 1 effective to reverse drug resistance will generally range from 15 mg/kg to 500 mg/kg. A unit dosage may contain from 25 to 500 mg of the oxazolone derivative, and can be taken one or more times per day. The oxazolone derivative can be administered with a

pharmaceutical carrier using conventional dosage unit forms either orally or parenterally.

Treatment of tumors with the medicaments prepared according to this invention requires that an anti-tumor effective amount of a chemotherapeutic aaent be administered together with a compound of formula 1. Tumors which can be treated with the medicaments prepared according to this invention include both benign and malignant tumors or neoplasms, and include melanomas, lymphomas, leukemias, and sarcomas. Illustrative examples of tumors are cutaneous tumors, such as malignant melanomas and mycosis fungoides; hematologic tumors such as leukemias, for example, acute lymphoblastic, acute myelocytic or chronic myelocytic leukemia; lymphomas, such as Hodgkin's disease or malignant lymphoma; gynecologic tumors, such as ovarian and uterine tumors; urologic tumors, such as those of the prostate, bladder or testis; soft tissue sarcomas, osseus or non-osseus sarcomas, breast tumors; tumors of the pituitary, thyroid and adrenal cortex; gastrointestinal tumors, such as those of the esophagus, stomach, intestine and colon; pancreatic and hepatic tumors; laryngeal papillomatosis and lung tumors. Of course those tumors which typically are or become multi-drug resistant are most beneficially treated with the medicaments prepared according to this invention. Such tumors include colon tumors, lung tumors, stomach tumors, and liver tumors.

The chemotherapeutic agents used together with the quinolyl- and isoquinolyloxazol-2-ones of formula I are those cytotoxic agents commonly used in the treatment of tumors. Illustrative examples of chemotherapeutic agents are: cyclophosphamide, methotrexate, prednisone, 6-mercaptopurine, procarbazine, daunorubicin, vincristine, vinblastine, chlorambucil, cytosine arabinoside, 6-thioguanine, thio TEPA, 5-fluorouracil, 5-fluoro-2-deoxyuridine, 5 azacytidine, nitrogen mustard, 1,3-bis(2-chloroethyl)-1-nitrosourea (BCNU), 1-(2-chloroethyl)-3-cyclohexyl-1-nitrosourea (CCNU), busulfan, adreamycin, bleomycin, vindesine, cycloleucine or methylglyoxal bis(guanylhydrazone) (i.e., MGBG). The effective amount of chemotherapeutic agent used with the medicaments prepared according to this invention varies widely and depends on factors such as the patient, the tumor tissue type and its size, and the particular chemotherapeutic agent selected. The amount is any effective amount and can be readily determined by those skilled in the art. In general, less chemotherpeutic agent will be required when administered with the oxazolones of formula 1, primarily because the problem of drug resistance need not addressed by the addition of larger quantities of chemotherapeutic agent. Of course mixtures of chemotherapeutic agents may be employed and surgical excission and radiation therapy may be useful adjuvants as in any tumor therapy. While the compound of formula 1 and the chemotherapeutic agent are said to be administered together, this does not necessarily mean that the compounds are formulated into the same dosage form or are administered concurrently. Rather, the expression "together" means that a compound of formula 1 and the chemotherapeutic agent(s) are administered in a combined dosage form or separately during the course of therapy.

The preferred route of administration is oral administration. For oral administration the oxazolone derivative can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds used according to this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptable surfactant, suspending agent, or emulsifying agent.

The oxazolone derivatives used according to this invention may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethylene-glycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutically adjuvants. Illustrative of oils which can be used in the parenteral formulations prepared according to this invention are those of petroleum, animal, vegetable, or synthetic origin, for example, peanut oil, soybean

oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions prepared according to this invention will typically contain from about 0.5 to about 25% by weight of the oxazolone derivative of formula 1 in solution. Preservatives and buffers may also be used advantageously. In order to minimize or eliminate irritation at the site of injection, such compositions may contain a non-ionic surfactant having a hydrophile-lipophile balance (HLB) of from 12 to 17. The quantity of surfactant in such formulations ranges from 5 to 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The following specific examples are presented to illustrate the synthesis of the compounds used according to this invention.

## EXAMPLE 1

### 1-Butyl-4-Quinoline Methanol (III)

In a 1 liter, 3-necked flask equipped with a mechanical stirrer, dropping funnel, reflux condenser (all dried under argon), and thermometer, were placed 15.0 grams (0.0954 mol) of 4-quinoline aldehyde and 400 ml of dry tetrahydrofuran (THF). The mixture was cooled by means of stirring in a dry ice/methanol bath to -70°C. Butylmagnesium chloride (100 ml of 2 M) was added through the funnel at a fast drop rate over a period of about 45 minutes, and the mixture was allowed to stir at -70°C for about an hour. Then, 100 ml saturated ammonium chloride ($NH_4Cl$) was added dropwise through the funnel and the mixture was allowed to warm to room temperature whereupon the resulting semi-solid material was filtered off under vacuum and washed with about 100 ml THF. The THF layers were combined and washed with saturated sodium chloride solution and then dried over magnesium sulfate. The inorganic matter was filtered off by vacuum through diatomaceous earth and the solvent evaporated. The residue was flash chromatographed on silica (1:1 ethyl acetate/hexane) and, after evaporation of the solvent, about 5.0 gram of purified title compound was recovered.

## EXAMPLE 2

### 1-(4-Quinolinyl)-1-Pentanone (IV)

In a 1 liter, 3-necked flask equipped with a mechanical stirrer, dropping funnel, reflux condenser (all dried under argon), and thermometer, were placed 50 ml of dry dichloromethane and 3.79 ml (0.043 mol) oxalyl chloride. The resulting mixture was stirred in a dry ice/methanol bath to maintain a temperature of -70°C. Methyl sulfoxide (6.17 ml, 0.043 mol) was added dropwise and subsequently a solution of 9.26 grams (0.043 mol) of the compound of Example 1 in dry dichloromethane ($CH_2Cl_2$) was added and the mixture allowed to stir cold for about 15 minutes. Triethylamine (35.6 ml) was then added and the mixture was allowed to stir cold for about 1 hour. After the mixture had been allowed to warm to room temperature, it was poured into a flask containing about 600 ml water. The $CB_2Cl_2$ layer was separated and the aqueous layer extracted with $CH_2Cl_2$ (2 times, 100 ml each). The combined $CH_2Cl_2$ layers were washed with saturated sodium chloride and dried over magnesium sulfate. The inorganic matter was filtered off and the solvent evaporated leaving a residue that was flash chromatographed as in Example 1. Evaporation left 9.0 grams of title compound.

## EXAMPLE 3

### 1-(4-Quinolinyl)-1-Pentanone Oxime (V)

In a 500 ml, 3-necked flask equipped with a mechanical stirrer, dropping funnel, reflux condenser (all dried under argon), were placed 8.3 grams (0.03892 mol) of the compound of Example 2, 4.12 grams (0.0584 mol)

of hydroxylamine hydrochloride, 40 ml of dry pyridine, and about 200 ml of dry ethanol. The mixture was refluxed for 6 hours, then the solvent was evaporated leaving a residue which was treated with about 400 ml ether and 200 ml water. The ether layer was separated and washed several times with water, washed with saturated sodium chloride and dried over magnesium sulfate. The inorganic matter was filtered off and the solvent evaporated, leaving 8.42 grams (94.7%) of the title compound.

**EXAMPLE 4**

**1-(4-Quinolinyl)-1-Pentanone-O-[(4-Methylphenyl)Sulfonyl] Oxime (VI)**

In a 250 ml erlenmeyer flask filled with argon were placed 8.42 grams (0.0369 mol) of the compound of Example 3 and about 130 ml dry $CH_2Cl_2$. While stirring and cooling to about 0°C in an ice/methanol bath, about 20 ml of triethylamine was added over a 5 minute period, then 10.55 grams (0.0554 mol) toluenesulfonyl chloride was added and the mixture allowed to stir for 3 hours. The solution was then evaporated to dryness which left a residue that was treated with ether and water. The ether phase was separated and the water phase extracted twice more with ether. The combined ether layers were extracted with dilute sodium hydroxide, washed with saturated sodium chloride and dried over magnesium sulfate. The inorganic matter was filtered off using vacuum, and the solvent was evaporated leaving 15.1 grams of the title compound.

**EXAMPLE 5**

**2-Amino-1-(4-Quinolinyl)-1-Pentanone (VII)**

In a 250 ml, 3-necked flask equipped with a mechanical stirrer, dropping funnel, reflux condenser (all dried under argon), was placed 60 ml dry ethanol. While stirring, 2.55 grams (0.111 mol) of sodium spheres were added and allowed to continue to stir under argon until the sodium dissolved. A solution of 15.1 grams of the compound of Example 4 in ethanol was then added and the mixture stirred for 4 hours at room temperature. The mixture was then poured into a flask containing 1200 ml absolute ether. The resulting precipitate was filtered off under vacuum through diatomaceous earth, and the filtrate extracted with 2N hydrochloric acid (3 times, 170 ml each). The extract was evaporated leaving 19.8 grams of the title compound.

**EXAMPLE 6**

**N-[2-Oxo-1-Propyl-2-(4-Quinolinyl)Ethyl]-1H-Imidazole-1-Carboxamide ($VIII_b$)**

The compound of Example 5 (19.8 grams) was dissolved in about 300 ml water, and the solution filtered by gravity into a 1 liter, 3-necked flask equipped with a mechanical stirrer and a thermometer. The solution was cooled to 0°C with stirring in an ice/methanol bath, and 29.87 grams (0.185 mol) 1,1'-carbonyldiimidazole was added over a 5 minute period. The mixture was allowed to stir cold for about 15 minutes. The resulting precipitate was taken up in about 500 ml ethyl acetate and separated from the water. The solution was washed with saturated sodium chloride and dried over magnesium sulfate, and the inorganic matter filtered off using diatomaceous earth under vacuum. The solvent was evaporated leaving the title compound.

**EXAMPLE 7**

**4-Propyl-5-(4-Quinolinyl)-1-(3H)-Oxazolone (I)**

The compound of Example 6 (12 grams) was heated under vacuum to 170°C for about 30 minutes, allowed to cool to room temperature and washed with water. The water was decanted and the residue was treated with $CH_2Cl_2$ (20 ml). The $CH_2Cl_2$ was evaporated leaving 7.8 grams of residue. The product was purified by means of flash chromatography on silica, eluting with ethyl acetate. The solvent was evaporated and the residue dissolved in 48 ml hot 50% ethanol, filtered and allowed to cool to room temperature. The precipitate was collected by vacuum filtration and dried *in vacuo* at 78°C, leaving 1.97 grams (21%) title compound. M.p. 188-190°C dec.; analysis calced. for $C_{15}H_{14}N_2O_2$: C, 70.85; H, 5.55; N, 11.02; analysis found: C, 71.10; H, 5.73; N, 10.76.

## EXAMPLE 8

### 1-(3-Quinolinyl)-1-butanone

In a dry 3-necked flask under argon at -50°C, n-butyl lithium (0,0025 mol, 0.021 ml) was added to 150 ml diethylether. Then 4.16 grams 3-bromoquinoline in 2 ml THF was added dropwise while stirring and maintaining the temperature at -60°C to -55°C. The solution was stirred for 30 minutes, and 2.3 grams N-methyl-N-methoxybutyramide were then added dropwise at -50°C and the solution was stirred an additional 30 minutes. The solution was then allowed to warm to 0°C and stirred for one hour. The reaction was quenched with a saturated solution of ammonium chloride and the THF layer separated, washed with brine, separated, and dried over magnesium sulfate. Filtration through diatomaceous earth, followed by concentration and subsequent thin layer chromatography (35% ethylacetate/65% $CH_2Cl_2$) gave a total yield of 2.03 g (51%) of the title compound.

By substituting the following starting materials for the 4-quinoline aldehyde and/or the butylmagnesium chloride of Example 1, and following the procedures set forth in Examples 1 through 7, the following end products can be made in a like manner.

A. 6- or 8-methoxy-4-quinoline aldehyde and methylmagnesium chloride, to yield 5-(6- or 8-methoxy-4-quinolinyl)-(3H)-oxazole-2-one

B. 2-quinoline aldehyde and methylmagnesium chloride, to yield 5-(2-quinolinyl)-(3H)-oxazole-2-one

C. 7- or 8-chloro-4-quinoline aldehyde and propylmagnesium chloride, to yield 4-ethyl-5-(7- or 8-chloro-4-quinolinyl)-(3H)-oxazole-2-one

D. 6,8-dichloro or dibromo-4-quinoline aldehyde and butylmagnesium chloride, to yield 4-propyl-5-(6,8-dichloro or dibromo-4-quinolinyl)-(3H)-oxazole-2-one

E. 7- or 8-nitro-4-quinoline aldehyde and pentylmagnesium chloride, to yield 4-butyl-5-(7- or 8-nitro-4-quinolinyl)-(3H)-oxazole-2-one

F. 7-trifluoromethyl-4-quinoline aldehyde and hexylmagnesium chloride, to yield 4-pentyl-5-(7-trifluoromethyl-4-quinolinyl)-(3H)-oxazole-2-one

G. 5,8-dimethoxy-4-quinoline aldehyde and benzylmagnesium chloride, to yield 4-phenyl-5-(5,8-dimethoxy-4-quinolinyl)-(3H)-oxazole-2-one

H. 5,8-dimethoxy-6-nitro-4-quinoline aldehyde and ethylmagnesium chloride, to yield 4-methyl-5-(5,8-dimethoxy-6-nitro-4-quinolinyl)-(3H)-oxazole-2-one

I. 6-methoxy-8-nitro-4-quinoline aldehyde and propylmagnesium chloride, to yield 4-ethyl-5-(6-methoxy-8-nitro-4-quinolinyl)-(3H)-oxazole-2-one

J. 5,6-dimethoxy-8-nitro-4-quinoline aldehyde and pentylmagnesium chloride, to yield 4-butyl-5-(5,6-dimethoxy-8-nitro-4-quinolinyl)-(3H)-oxazole-2-one

K. 5-methyl-4-quinoline aldehyde and benzylmagnesium chloride, to yield 4-phenyl-5-(5-methyl-4-quinolinyl)-(3H)-oxazole-2-one

L. 2-(4-methoxy)phenyl-4-quinoline aldehyde and 3,5-dimethoxybenzylmagnesium chloride, to yield 4-(3,5-dimethoxyphenyl)-5-[1-(4-methoxyphenyl)-4-quinolinyl]-(3H)-oxazole-2-one

M. 6,8-dimethoxy-4-quinoline aldehyde and 4-methylbenzylmagnesium chloride, to yield 4-(4-methylphenyl)-5-(6,8-dimethoxy-4-quinolinyl)-(3H)-oxazole-2-one

N. 4-quinoline aldehyde and propylmagnesium chloride, to yield 4-ethyl-5-(4-quinolinyl)(3H)-oxazole-2-one (mp 273-76°C)

O. 4-quinoline aldehyde and pentylmagnesium chloride, to yield 4-butyl-5-(4-quinolinyl)(3H)-oxazole-2-one (mp 196-98°C)

P. 3-quinoline aldehyde and butylmagnesium chloride, to yield 4-propyl-5-(3-quinolinyl)(3H)-oxazole-2-one (mp 193-95°C)

Q. 4-isoquinoline aldehyde and butylmagnesium chloride to yield 4-propyl-5-(4-isoquinolinyl)(3H)-oxazole-2-one (mp 122-24°C).

By substituting 1-(3-quinolinyl)-1-pentanone for the compound of Example 2 and by following the procedure set forth in Examples 3 through 7, 4-propyl-5-(3-quinolinyl)-(3H)-oxazolone is produced.

In a like manner, by substituting the following starting materials for 1-(4-quinolinyl)-1-pentanone of Example 2 and following the procedure set forth in the preceding paragraph, the following end products can be made.

R. 1-(5-quinolinyl)-1-pentanone, to yield 4-propyl-5-(5-quinolinyl)-(3H)-oxazole-2-one

S. 1-(6-quinolinyl)-1-butanone, to yield 4-ethyl-5-(6-quinolinyl)-(3H)-oxazole-2-one

T. 1-(7-quinolinyl)-1-ethanone, to yield 5-(7-quinolinyl)-(3H)-oxazole-2-one

U. 1-(8-quinolinyl)-1-phenylethanone, to yield 4-phenyl-5-(8-quinolinyl)-(3H)-oxazole-2-one

V. 1-(5,8-dimethoxy-3-quinolinyl)-1-propanone, to yield 4-methyl-5-(5,8-dimethoxy-3-quinolinyl)-(3H)-oxazole-2-one.

The following specific examples are presented to illustrate compositions of this invention, but they should not be construed as limiting the scope of this invention in any way.

**EXAMPLE 9**

A tablet is prepared from

| | |
|---|---|
| 4-Methyl-5-(3-quinolinyl)-(3H)-oxazole-2-one | 250 mg |
| Starch | 40 mg |
| Talc | 10 mg |
| Magnesium | 10 mg |

**EXAMPLE 10**

A capsule is prepared from

| | |
|---|---|
| 4-phenyl-5-(2-quinolinyl)-(3H)-oxazole-2-one | 400 mg |
| Talc | 40 mg |
| Sodium Carboxymethylcellulose | 40 mg |
| Starch | 120 mg |

## Claims

1. Use of compounds of formula I

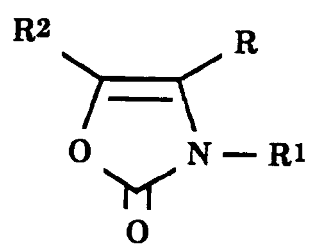

(I)

wherein

R and $R^1$ are each independently selected from hydrogen, $C_1$-$C_6$ alkyl, and phenyl or $C_1$-$C_3$ alkylphenyl wherein the phenyl ring is optionally substituted with one, two or three of the substituents selected from fluorine, chlorine, bromine, $C_1$-$C_4$ alkyl, and $C_1$-$C_4$ alkoxy; and

$R^2$ is a 2-, 3-, or 4-quinolyl group or 1-,3-, or 4-isoquinolyl group wherein the quinolyl or isoquinolyl group is optionally substituted with one, two or three of the substituents selected from fluorine, chlorine, bromine, $C_1$-$C_4$ alkyl, $C_1$-$C_4$ alkoxy, nitro and trifluoromethyl or phenyl wherein the phenyl is optionally substituted by fluorine, chlorine, bromine, $C_1$-$C_4$ alkyl, or $C_1$-$C_4$ alkoxy; or

$R^2$ is a 5-, 6-, 7-, or 8-quinolyl or isoquinolyl group;

and the pharmaceutically acceptable salts thereof for the production of a medicament for the treatment of a drug resistant tumor.

2. The use according to Claim 1 wherein the medicament is combined with a anti-tumor effective amount of a chemotherapeutic agent.

3. The use according to Claim 1 or 2 wherein in the compound of the formula I $R^2$ is a 5-, 6-, 7-, or 8-quinolyl group.

4. The use according to any one of Claims 1 to 3 wherein in the compound of the Formula I $R^2$ is an optionally substituted 2-, 3-, or 4-quinolyl group.

5. The use according to Claim 4 wherein in the compound of the formula I R and $R^1$ are each independently selected from hydrogen or $C_1$-$C_6$ alkyl.

6. The use according to Claim 5 wherein in the compound of the formula I R is $C_1$-$C_6$ alkyl and $R^1$ is hydrogen.

7. The use according to Claim 4 wherein in the compound of the formula I $R^2$ is an unsubstituted 2-, 3-, or 4-quinolyl group.

8. The use according to Claim 7 wherein in the compound of the formula I $R^2$ is 4-quinolinyl, R is propyl, and $R^1$ is hydrogen.

9. The use according to any one of claims 1 to 8 wherein the tumor is a tumor of the colon, lung, liver or stomach.

10. The use according to any one of claims 2 to 8 wherein the chemotherapeutic agent is daunorubicin, vincristine, vinblastine, bleomycin, or adriamycin.

## Patentansprüche

1. Verwendung von Verbindungen der Formel I

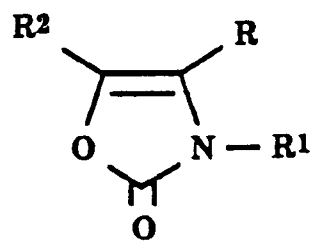

(I)

wobei

R und $R^1$ jeweils unabhängig voneinander Wasserstoffatome, $C_1$-$C_6$-Alkylreste, Phenylgruppen oder $C_1$-$C_3$-Alkylphenylreste sind, wobei der Phenylring gegebenenfalls mit einem, zwei oder drei Fluor-, Chlor-, Bromatomen, $C_1$-$C_4$-Alkyl- oder $C_1$-$C_4$-Alkoxyresten substituiert ist; und

$R^2$ eine 2-, 3- oder 4-Chinolylgruppe oder eine 1-, 3- oder 4-Isochinolylgruppe ist, die gegebenenfalls mit einem, zwei oder drei Fluor-, Chlor-, Bromatomen, $C_1$-$C_4$-Alkyl-, $C_1$-$C_4$-Alkoxyresten, Nitrogruppen, Trifluormethylgruppen oder Phenylgruppen substituiert sind, wobei die Phenylgruppe gegebenenfalls mit Fluor-, Chlor-, Bromatomen, $C_1$-$C_4$-Alkyl- oder $C_1$-$C_4$-Alkoxyresten substituiert ist; oder

$R^2$ eine 5-, 6-, 7- oder 8-Chinolyl- oder Isochinolylgruppe ist;

und der pharmazeutisch verträglichen Salze davon zur Herstellung eines Arzneimittels zur Behandlung eines gegen Arzneistoffe resistenten Tumors.

2. Verwendung nach Anspruch 1, wobei das Arzneimittel mit einer gegen Tumore wirksamen Menge eines chemotherapeutischen Mittels kombiniert wird.

3. Verwendung nach Anspruch 1 oder 2, wobei in der Verbindung der Formel I $R^2$ eine 5-, 6-, 7- oder 8-Chinolylgruppe ist.

4. Verwendung nach einem der Ansprüche 1 bis 3, wobei in der Verbindung der Formel I $R^2$ eine gegebenenfalls substituierte 2-, 3- oder 4-Chinolylgruppe ist.

5. Verwendung nach Anspruch 4, wobei in der Verbindung der Formel I R und $R^1$ jeweils unabhängig voneinander ein Wasserstoffatom oder ein $C_1$-$C_6$-Alkylrest sind.

6. Verwendung nach Anspruch 5, wobei in der Verbindung der Formel I R ein $C_1$-$C_6$-Alkylrest und $R^1$ ein Wasserstoffatom ist.

7. Verwendung nach Anspruch 4, wobei in der Verbindung der Formel I $R^2$ eine unsubstituierte 2-, 3- oder 4-Chinolylgruppe ist.

8. Verwendung nach Anspruch 7, wobei in der Verbindung der Formel I $R^2$ eine 4-Chinolinylgruppe, R eine Propylgruppe und $R^1$ ein Wasserstoffatom ist.

9. Verwendung nach einem der Ansprüche 1 bis 8, wobei der Tumor ein Dickdarm-, Lungen-, Leber- oder Magentumor ist.

10. Verwendung nach einem der Ansprüche 2 bis 8, wobei das chemotherapeutische Mittel Daunorubicin, Vincristin, Vinblastin, Bleomycin oder Adriamycin ist.

## Revendications

1. Utilisation des composés de formule (I) :

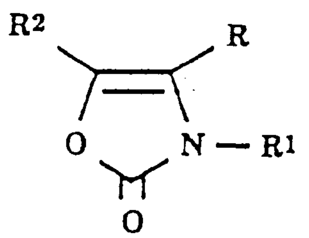

(I)

dans laquelle :

R et $R^1$ sont chacun choisis indépendamment parmi les suivants : un atome d'hydrogène, un groupe $C_1$-$C_6$ alkyle, et phényle ou $C_1$-$C_3$ alkylphényle dans lequel le noyau phényle est éventuellement substitué par 1, 2 ou 3 substituants choisis parmi les suivants : fluor, chlore, brome, $C_1$-$C_4$-alkyle et $C_1$-$C_4$ alcoxy ; et

$R^2$ est un groupe 2-, 3- ou 4-quinolyle ou un groupe 1-, 3- ou 4-isoquinolyle dans lequel le groupe quinolyle ou isoquinolyle est éventuellement substitué par 1, 2 ou 3 des substituants choisis parmi les suivants : fluor, chlore, brome, $C_1$-$C_4$-alkyle, $C_1$-$C_4$-alcoxy, nitro et trifluorométhyle ou phényle dans lequel le groupe phényle est éventuellement substitué par l'un des suivants : fluor, chlore, brome, $C_1$-$C_4$-alkyle ou $C_1$-$C_4$-alcoxy ; ou bien

$R^2$ est un groupe 5-, 6-, 7- ou 8-quinolyle ou isoquinolyle ;

et les sels pharmaceutiquement acceptables de ces composés pour la production d'un médicament pour le traitement d'une tumeur résistant aux médicaments.

2. L'utilisation selon la revendication 1, selon laquelle le médicament est combiné ensemble avec une quantité antitumorale efficace d'un agent chimiothérapeutique.

3. L'utilisation selon la revendication 1 ou 2, selon laquelle dans le composé de formule (I) $R^2$ est un groupe 5-, 6-, 7- ou 8-quinolyle.

4. L'utilisation selon l'une quelconque des revendications 1 à 3, selon laquelle dans le composé de formule (I) $R^2$ est un groupe 2-, 3- ou 4-quinolyle éventuellement substitué.

5. L'utilisation selon la revendication 4 selon laquelle dans le composé de formule (I) R et $R^1$ sont chacun choisi indépendamment parmi l'hydrogène et un groupe $C_1$-$C_6$-alkyle.

**6.** L'utilisation selon la revendication 5, selon laquelle dans le composé de formule (I) R est un groupe $C_1$-$C_6$-alkyle et $R^1$ est un atome d'hydrogène.

**7.** L'utilisation selon la revendication 4, selon laquelle dans le composé de formule (I) $R^2$ est un groupe 2-, 3- ou 4-quinolyle non substitué.

**8.** L'utilisation selon la revendication 7, selon laquelle dans le composé de formule (I) $R^2$ est un groupe 4-quinolinyle, R est un groupe propyle et $R^1$ est un atome d'hydrogène.

**9.** L'utilisation selon l'une quelconque des revendications 1 à 8, selon laquelle la tumeur est une tumeur du colon, des poumons, du foie ou de l'estomac.

**10.** L'utilisation selon l'une quelconque des revendications 2 à 8, selon laquelle l'agent chimiothérapeutique est la daunorubicine, la vincristine, la vinblastine, la bléomycine ou l'adriamycine.